(19) Europäisches Patentamt
European Patent Office
Office européen des brevets

(11) EP 1 270 568 A1

(12) **EUROPEAN PATENT APPLICATION**

(43) Date of publication:
**02.01.2003 Bulletin 2003/01**

(51) Int Cl.[7]: **C07D 409/04**, A61K 31/4436, A61P 25/06

(21) Application number: **01114643.8**

(22) Date of filing: **19.06.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Biofrontera Pharmaceuticals AG
51377 Leverkusen (DE)**

(72) Inventors:
- **Bellott, Emile, Ph.D.
Beverly, MA 01915 (US)**
- **Froimowitz, Mark, Ph.D.
Newton Centre, MA 02459 (US)**
- **Gordon, Douglas, Ph.D.
Burlington, MA 01803 (US)**
- **Lübbert, Hermann, Prof. Dr.
51381 Leverkusen (DE)**
- **Ullmer, Christoph, Dr.
51467 Bergisch Gladbach (DE)**

(74) Representative:
**Sternagel, Fleischer, Godemeyer & Partner
Patentanwälte
Braunsberger Feld 29
51429 Bergisch Gladbach (DE)**

(54) **1-methyl-4-(3-ethoxy-9h-thioxanthene-ylidene)-piperidine and its use as 5-HT2B and/or H1 antagonist**

(57) The present invention relates to 1-methyl-4-(3-ethoxy-9H-thioxanthene-9-ylidene)-piperidine and pharmaceutically acceptable salts thereof, use of 1-methyl-4-(3-ethoxy-9H-thioxanthene-9-ylidene)-piperidine as a medicament and for the manufacture of a medicament for treatment of a disease state which is alleviable by treatment with a 5-$HT_{2B}$ or $H_{1o}$ or 5-$HT_{2B}$/$H_1$ receptor antagonists.

EP 1 270 568 A1

Printed by Jouve, 75001 PARIS (FR)

## Description

The present invention relates to 1-methyl-4-(3-ethoxy-9H-thioxanthene-9-ylidene)-piperidine and pharmaceutically acceptable salts thereof, which exhibit useful pharmacological properties, including utility as 5-$HT_{2B}$ or $H_{1\ o}$ or 5-$HT_{2B}$/$H_1$ receptor antagonists for treatment of a disease state which is alleviable by treatment with a 5-$HT_{2B}$ or $H_{1\ o}$ or 5-$HT_{2B}$/$H_1$ receptor antagonist.

Serotonin, a neurotransmitter with mixed and complex pharmacological characteristics, was first discovered in 1948, and subsequently has been the subject of substantial research. Serotonin, also referred to as 5-hydroxytryptamine (5-HT), acts both centrally and peripherally on discrete 5-HT receptors. Currently, fourteen subtypes of serotonin receptor are recognized and delineated into seven families, 5-$HT_1$, to 5-$HT_7$. Within the 5-$HT_2$ family, 5-$HT_{2A}$, 5-$HT_{2B}$ and 5-$HT_{2C}$ subtypes are known to exist. These subtypes share sequence homology and display similarities in their specificity for a wide range of ligands. Nomenclature and classification of 5-HT receptors have been reviewed recently (see Martin and Humphrey, Neuropharm. 1994, 33, 261-273 and Hoyer et al., Pharm. Rev. 1994, 46, 157-203).

The 5-$HT_{2B}$ receptor, initially termed 5-$HT_{2F}$, or serotonin-like receptor, was first characterized in rat isolated stomach fundus (see Clineschmidt et al., J. Pharmacol. Exp. Ther. 1985, 235, 696-708; Cohen and Wittenauer, J. Cardiovasc. Pharmacol. 1987, 10, 176-181) and initially cloned from rat (see Foguet et al., EMBO 1992, 11, 3481-3487) followed by the cloning of the human 5-$HT_{2B}$ receptor (see Schmuck et al., FEBS Lett. 1994, 342, 85-90; Kursar et al., Mol. Pharmacol. 1994, 46, 227-234). The 5-$HT_{2C}$ receptor, widely distributed in the human brain, was first characterized as a 5-$HT_{1C}$ subtype (see Pazos et al., Eur. J.

Pharmacol. 1984, 106, 539-546) and was subsequently recognized as belonging to the 5-$HT_2$ receptor family (see Pritchett et al., EMBO J. 1988, 7, 4135-4.140).

Because of the similarities in the pharmacology of ligand interactions at 5-$HT_{2B}$ and 5-$HT_{2C}$ receptors, many of the therapeutic targets that have been proposed for 5-$HT_{2C}$ receptor antagonists are also targets for 5-$HT_{2B}$ receptor antagonists. Current evidence strongly supports a therapeutic role for 5-$HT_{2B/2C}$ receptor antagonists in treating anxiety (e.g., generalized anxiety disorder, panic disorder and obsessive compulsive disorder), alcoholism and addiction to other drugs of abuse, depression, migraine, sleep disorders, feeding disorders (e.g., anorexia nervosa) and priapism. Additionally, current evidence strongly supports a therapeutic role for selective 5-$HT_{2B}$ receptor antagonists that will offer distinct therapeutic advantages collectively in efficacy, rapidity of onset and absence of side effects. Such agents are expected to be useful in the treatment of hypertension, disorders of the gastrointestinal track (e.g., irritable bowel syndrome, hypertonic lower esophageal sphinter, motility disorders), restenosis, asthma and obstructive airway disease, and prostatic hyperplasia (e.g., benign prostatic hyperplasia).

US-A-3,275,640 describes generically substituted 1-hydrocarbyl-4-(9H-thioxanthene-9-ylidene)-piperidines and their preparation. It is also disclosed that the compounds may be used as therapeutic agents because of their antihistaminic and/or antiserotonin properties.

US-A-3,557,287 relates to a combination preparation for use in the threatment of headaches of vascular origin containing as active constituents (a) a vasotonic lysergic acid selected from ergostine, ergotamine, dihydroergostine, dihydroergotamine, ergovaline, 5'-methylergoalanine; (b) caffeine; and (c) 9-(1-methyl-4-perperidylidene)thioxanthene (= 1-methyl-4-(9H-thioxanthene-9-ylidene)-piperidine.

In DE-A-22 56 392 discloses 4-(9H-thioxanthene-9-ylidene)-piperidine derivatives wherein the nitrogen atom of the piperidine ring is bonded to an alkyl radical substituted with cyano, -COR or -COOR. Sleep-inducing properties are attributed to these derivatives.

JP-A-61106573 refers to the use of substituted 4-(9H-thioxanthene-9-ylidene)-piperidines as pesticides.

It is the object of the present invention to provide a compound acting as selective 5-$HT_{2B}$ or $H_{1\ o}$ or 5-$HT_{2B}$/$H_1$ receptor antagonist.

The object is met by 1-methyl-4-(3-ethoxy-9H-thioxanthene-9-ylidene)-piperidine according to formula

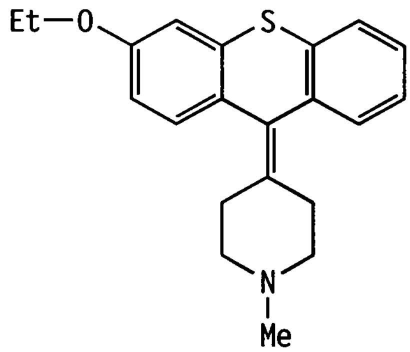

wherein Et represents an ethyl radical and Me represents a methyl radical, or a pharmaceutically acceptable salt thereof.

The present invention is also directed to the use of 1-methyl-4-(3-ethoxy-9H-thioxanthene-9-ylidene)-piperidine for the manufacture of a medicament for treatment of a disease state which is alleviable by treatment with a 5-$HT_{2B}$ or $H_{1\ o}$ or 5-$HT_{2B}/H_1$receptor antagonist.

The present invention further relates to a pharmaceutical composition comprising 1-methyl-4-(3-ethoxy-9H-thioxanthene-9-ylidene)-piperidine or a pharmaceutically acceptable salt thereof, in admixture with one or more pharmaceutically acceptable carriers.

"Pharmaceutically acceptable salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

The term "treatment" as used herein covers any treatment of a disease in a mammal, particularly a human, and includes:

(i) preventing the disease from occurring in a subject which may be predisposed to the disease but has not yet been diagnosed as having it;
(ii) inhibiting the disease, i.e., arresting its development; or
(iii) relieving the disease, i.e., causing regression of the disease.

The term "disease state which is alleviable by treatment with a 5-$HT_{2B}$ or $H_{1\ o}$ or 5-$HT_{2B}/H_1$receptor antagonist" as used herein is intended to cover all disease states which are generally acknowledged in the art to be usefully treated with compounds having affinity for 5-$HT_{2B}$ or $H_{1\ o}$ or 5-$HT_{2B}/H_1$ receptors in general, and those disease states which have been found to be usefully treated by 1-methyl-4-(3-ethoxy-9H-thioxanthene-9-ylidene)-piperidine. Such disease states include, but are not limited to, migraine, pain (e.g. acute, chronic, neuropathic, inflammatory and cancer pain) hypertension, disorders of the gastrointestinal track (e.g., irritable bowel syndrome, hypertonic lower esophageal sphinter, motility disorders), restenosis, asthma and obstructive airway disease, prostatic hyperplasia (e.g., benign prostatic hyperplasia), and priapism, seasonal and perennial rhinitis, the symptoms of allergic asthma, chronic idiopathic urticaria, some physical urticaria

Although US-A-3,275,640 describes the antihistaminic and/or antiserotonin properties of the class of substituted 1-hydrocarbyl-4-(9H-thioxanthene-9-ylidene)-piperidines in general it neither discloses the 4-(9H-thioxanthene-9-ylidene)-piperidine derivative of the present invention nor correlates a selected derivative with a special antihistaminic and/or antiserotonin property. The term "antiserotonin property" is indeed a very broad term and refers to 14 different receptor subtypes. Even more US-A-3,275,640 does not give any hint that a member of the class of substituted 1-hydrocarbyl-4-(9H-thioxanthene-9-ylidene)-piperidines has a selective affinity for one of the various 5-HT receptor subtypes, namely the human 5-$HT_{2B}$ receptor.
The term "antihistaminergic properties" is again a broad term and refers to 4 different receptor subtypes. US-A-3,275,640 does not give any hint that a member of the class of substituted 1-hydrocarbyl-4-(9H-thioxanthene-9-ylidene)-piperidines has a selective affinity for one of the various Histamine receptor subtypes, namely the human $H_1$ receptor.

The property of the novel 4-(9H-thioxanthene-9-ylidene)-piperidine derivative as a selective 5-$HT_{2B}/H_1$ re-

ceptor antagonist provides the possibility for a more specific treatment of the above-cited disease states and reduction of eventually undesired side effects at the same time.

A general method for preparing substituted 1-hydrocarbyl-4-(10-thioxanthylidene)-piperidines is described in US-A-3,275,640. The novel 1-methyl-4-(3-ethoxy-9H-thioxanthene-9-ylidene)-piperidine may be obtained according to that method. 3-Ethoxythioxanthone 1 is reacted with the Grignard reagent 2 prepared from 1-methyl-4-halopiperidine, preferably 1-methyl-4-chloropiperidine, according to Scheme I. The alcohol 3 is isolated and dehydrated with an acid, preferably hydrochloric acid or formic acid to produce 1-methyl-4-(3-ethoxy-9H-thioxanthene-9-ylidene)-piperidine 4.

Scheme I:

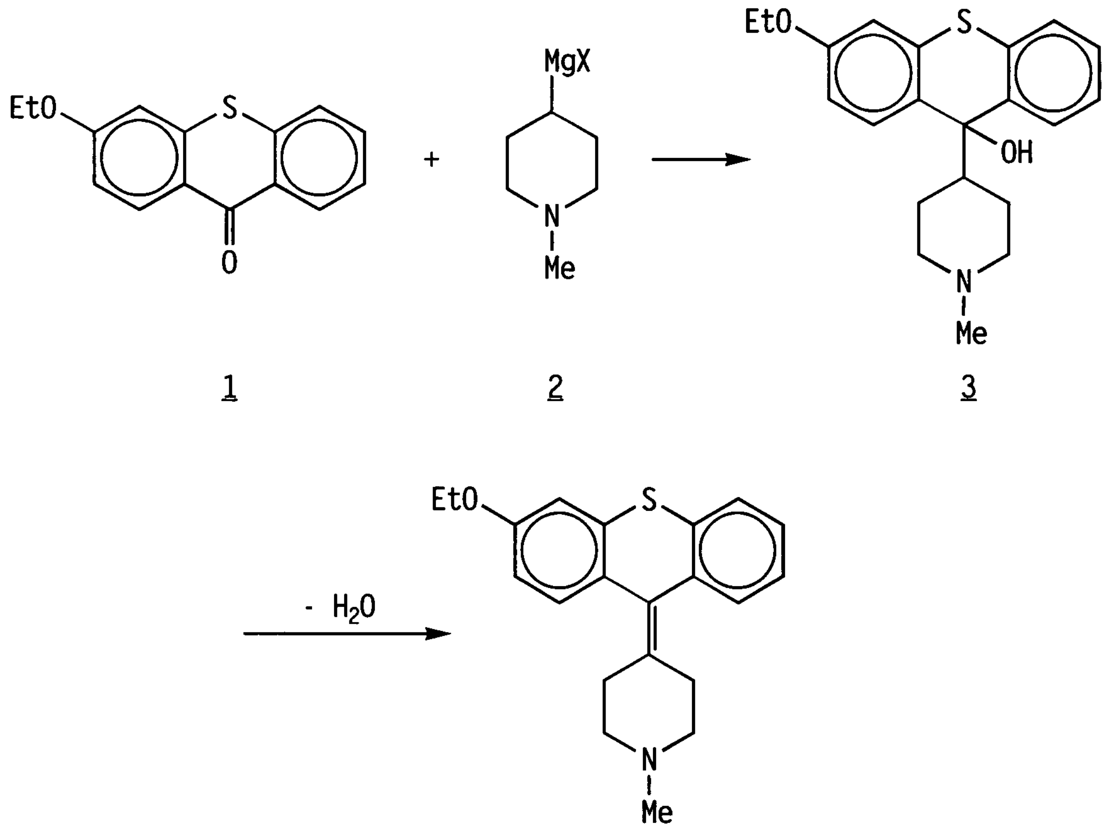


1 2 3

4

One way to synthesize the 3-ethoxythioxanthone educt 1 starts from 3-methoxythioxanthone 8a that may be prepared by a procedure described by I. Cervena, J. Metysova, E. Svatek, B. Kakac, J. Holubek, M. Hrubantova, and M. Protiva, in Coll. Czech. Chem. Comm. 41, 881-904 (1978) (Scheme II). 3-Methoxythiol 5 is reacted with 2-iodobenzoic acid 6 in a boiling solution of KOH in the presence of copper. After addition of hydrochloric acid the coupled acid 7 is obtained. The acid 7 is cyclized with polyphosphoric acid to produce a mixture of the isomeres 3-methoxythioxanthone 8a and 1-methoxythioxanthone 8b that can be separated by chromatography, preferably column chromatography.

Scheme II:

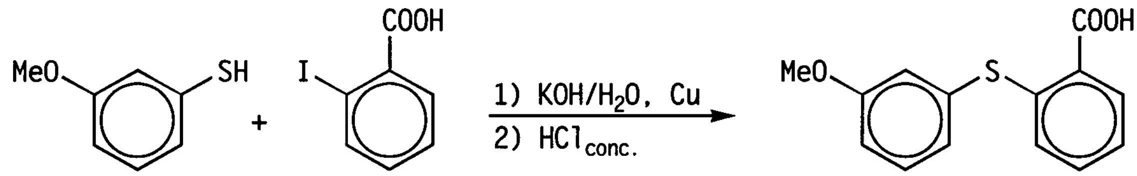


5 6 7

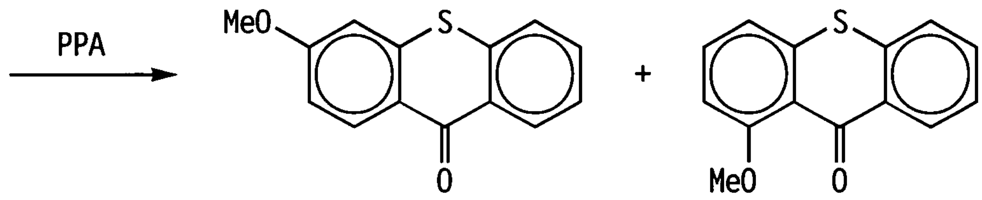


8a 8b

The separated 3-methoxythioxanthene 8a is first transferred to 3-hydroxy thioxanthene 9 by treatment with hydrobromic acid and acetic acid. The 3-hydroxy thioxanthene 9 is then reacted with iodoethane in the presence of a base, preferably $K_2CO_3$, to produce 3-ethoxythioxanthone 1 according to Scheme III:

Scheme III:

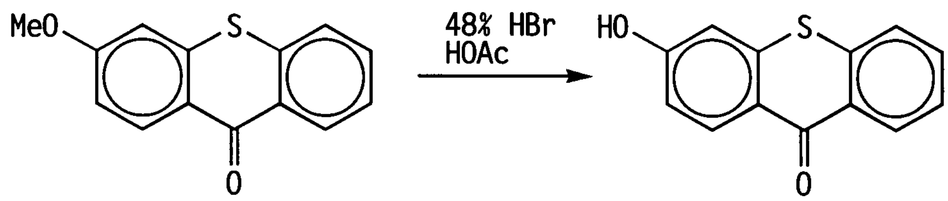


8a 9

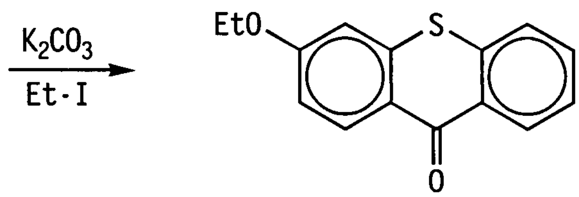


1

The compounds of this invention are selective human 5-$HT_{2B}$ or $H_{1\ o}$ or 5-$HT_{2B}$/$H_1$ receptor antagonists. Affinity for the 5-$HT_{2B}$ receptors was demonstrated using an in vitro binding assay utilizing cloned human 5-$HT_{2B}$ receptors radiolabelled with [$^3$H]-5HT, as shown in the examples. Selectivity for the human 5-$HT_{2B}$ receptor was shown by counter screening at human 5-$HT_{2A}$ and 5-$HT_{2C}$ receptors. Antagonist properties were determined in rat stomach fundus longitudinal muscle. Affinity for the human $H_1$ receptor was demonstrated using an in vitro binding assay utilizing cloned human $H_1$ receptors radiolabelled with [$^3$H]-mepyramine, as shown in the examples. Selectivity for the human $H_1$ receptor was shown by counter screening at human $H_2$, $H_3$ and $H_4$ receptors. Antagonist properties were determined by [$^3$H]Inositol phosphate production in transiently transfected HEK-293 cells.

Accordingly, the compounds of this invention are useful for treating diseases which can be ameliorated by blockade of 5-$HT_{2B}$ and/or $H_1$ receptors. Because of the similarities in the pharmacology of ligand interactions at 5-$HT_{2C}$ and 5-$HT_{2B}$ receptors many of the therapeutic targets that have been proposed for 5-$HT_{2C}$ receptor antagonists are also targets for 5-$HT_{2B}$ receptor antagonists. In particular, several clinical observations suggest a therapeutic role for 5-$HT_{2B}$ receptor antagonists in the prevention of migraine, in that mobilization of 5-HT into the plasma is believed to be a precipitating factor in migraine.
Additionally, non-selective 5-$HT_{2B}$ receptor agonists provoke migraine attacks in susceptible individuals, and non-selective 5-$HT_{2B}$ receptor antagonists are effective in preventing the onset of migraine (see Kalkman, Life Sciences 1994, 54, 641-644). It is speculated that activation of 5-$HT_{2B}$ receptors located on endothelial cells of meningeal blood vessels triggers migraine attacks through the formation of nitric oxide (see Schmuck et al., Eur. J. Neurosci. 1996, 8, 959-967).
Activation of human $H_1$ receptors induces a nitric oxide-mediated headache in healthy individuals (see Lassen et al., Neuroreport 1995, 31, 1475-1477. Analogously, human cerebral arteries have also been shown to express endothelial $H_1$ and $H_2$ receptors, the former subtype mediating vasodilatation (see Ottosson et al., Br. J. Pharmacol. 1988, 94, 901-907.

Experimental evidence indicates that the compound of the present invention are useful in the treatment of pain, including acute, chronic, neuropathic, inflammatory, and cancer pain, particularly inflammatory pain. 5-HT (serotonin) plays a key role in the regulation of transmission of nociceptive information at various levels of the peripheral and central nervous systems. (See Richardson, B. P., "Serotonin and Pain", Ann. N.Y. Acad. Sci., 1990, 600, 511-520). Moreover, neuronal systems counting 5-HT are involved not only in the regulation of nociceptive input at the spinal and supraspinal level, but in mediating the nociceptive action of other analgesics including the opiates. 5-HT is a mediator of sensitization of nerve terminal nociceptors that may occur in the genesis of pain associated with inflammation. The 5-$HT_{2B}$ receptor is highly sensitive to activation by 5-HT and specific blockade by selective 5-$HT_{2B}$ antagonists may provide a novel avenue toward analgesia therapy.

Experimental evidence supports a therapeutic role for 5-$HT_{2B}$ receptor antagonists in treating hypertension. In hypertension, one of the most profound increases in vascular responsiveness is observed for serotonin. Two lines of evidence imply that this results from a switch in the receptor mediating vasoconstriction from predominantly 5-$HT_{2A}$ to predominantly 5-$HT_{2B}$. First, serotonin induced contractions of isolated blood vessels from hypertensive animals become resistant to block by selective 5-$HT_{2A}$ receptor antagonists, but remain sensitive to non-selective 5-$HT_{2B}$ receptor antagonists. Second, there is an increase in 5-$HT_{2B}$ receptor mRNA in vessels from hypertensive animals (see Watts et al., J. Pharmacol. Exp. Ther. 1996, 277, 1103-13 and Watts et al., Hypertension 1995, 26, 1056-1059). This hypertension-induced shift in the population of receptor subtype mediating constrictor responses to 5-HT suggests that selective block of vasoconstrictor 5-$HT_{2B}$ receptors may be of therapeutic benefit in the treatment of hypertension.

Clinical and experimental evidence support a therapeutic role for 5-$HT_{2B}$ receptor antagonists in treating disorders of the gastrointestinal track, in particular irritable bowel syndrome (IBS). Although the pathology underlying IBS remains unclear, there is a well-established implied role for the involvement of serotonin. Thus, meals with a high serotonin content can exacerbate symptoms in some patients (see Lessorf, Scand. J. Gastroenterology 1985, 109, 117-121), while in pre-clinical studies, serotonin has been shown directly to sensitize visceral sensory neurons resulting in an enhanced pain response similar to that observed in IBS (see Christian et al., J. Applied Physiol. 1989, 67, 584-591 and Sanger et al., Neurogastroenterology and Motility 1996, 8, 319-331). The possibility that 5-$HT_{2B}$ receptors play a crucial role in the sensitizing actions of serotonin are suggested by several lines of evidence. Firstly, 5-$HT_{2B}$ receptors are present in the human intestine (see Borman et al., Brit. J. Pharmacol. 1995, 114, 1525-1527 and Borman et al., Ann. of the New York Acad. of Sciences 1997, 812, 222-223). Secondly, activation of 5-$HT_{2B}$ receptors can result in the production of nitric oxide, an agent capable of sensitizing sensory nerve fibers (see Glusa et al., Naunyn-Schmied. Arch. Pharmacol. 1993, 347, 471-477 and Glusa et al., Brit. J. Pharmacol. 1996, 119, 330-334). Thirdly, poorly selective drugs which display high affinity for the 5-$HT_{2B}$ receptor are clinically effective in reducing the pain associated with IBS and related disorders (see Symon et al., Arch. Disease in Childhood 1995, 72, 48-50 and Tanum et al., Scand. J. Gastroenterol. 1996, 31, 318-325). Together these findings suggest that a selective 5-$HT_{2B}$ receptor antagonist will attenuate both the gastrointestinal pain and abnormal motility associated with IBS.

Clinical and experimental evidence support a therapeutic role for 5-$HT_{2B}$ receptor antagonists in treating restenosis. Angioplasty and bypass-grafting are associated with restenosis which limits the efficacy of these procedures. Platelet-rich thrombus formation is the predominant cause of acute occlusion whereas serotonin, among other platelet-derived mediators, is thought to contribute to late restenosis (see Barradas et al., Clinica Chim. Acta 1994, 230, 157-167). This late restenosis involves proliferation of the vascular smooth muscle. Two lines of evidence implicate a role for 5-$HT_{2B}$ receptors in this process. Firstly, serotonin displays a potent mitogenic activity in cultured smooth muscle and endothelial cells via activation of 5-$HT_2$ receptors (see Pakala et al., Circulation 1994, 90, 1919-1926). Secondly, this mitogenic activity appears to be mediated via activation of a tyrosine kinase second messenger pathway involving mitogen activated protein kinase (MAPK) (see Lee et al., Am. J. Physiol. 1997, 272(1 pt 1), C223-230 and

Kelleher et al., Am. J. Physiol. 1995, 268(6 pt 1), L894-901). The recent demonstration that 5-$HT_{2B}$ receptors couple to MAPK (see Nebigil et al., Proc. Natl. Acad Sci. U.S.A. 2000, 97, 22591-2596), coupled with the high affinity of serotonin for this receptor subtype, indicates that a selective 5-$HT_{2B}$ receptor antagonist may afford protection against restenosis of autografted blood vessels or of vessels following angioplasty.

Clinical and experimental evidence support a therapeutic role for 5-$HT_{2B}$ receptor antagonists in treating asthma and obstructive airway disease. Abnormal proliferation of airways smooth muscle, together with hyper-reactivity of the smooth muscle to constrictor stimuli including serotonin, plays a significant role in the pathogenesis of human airway disease such as asthma and bronchial pulmonary dysplasia (see James et al., Am. Review of Respiratory Disease 1989, 139, 242-246 and Margraf et al., Am. Review of Respiratory Disease 1991, 143, 391-400). In addition to other subtypes of serotonin receptor, 5-$HT_{2B}$ receptors are present in bronchial smooth muscle (see Choi et al., Febs Letters 1996, 391, 45-51) and have been shown to stimulate smooth muscle mitogenesis in airways smooth muscle (see Lee et al., Am. J. Physiol. 1994, 266, L46-52). Since elevated concentrations of circulating free serotonin are closely associated with clinical severity and pulmonary function in symptomatic asthmatics, serotonin may play an important role in the pathophysiology of acute attacks (see Lechin et al., Ann. Allergy, Asthma, Immunol. 1996, 77, 245-253). These data suggest that an antagonist of 5-$HT_{2B}$ receptors in airways smooth muscle may therefore be useful in preventing airways constriction resulting from the elevated levels of circulating serotonin and prevent proliferation of the airways smooth muscle that contributes to the long-term pathology of this disease.

Experimental evidence supports a therapeutic role for 5-$HT_{2B}$ receptor antagonists in treating prostatic hyperplasia. Obstruction of the urinary tract can occur as a result of prostatic hyperplasia and excessive prostatic constriction of the urethra. This in turn leads to diminished urinary flow rates and an increased urgency and frequency of urination. 5-$HT_{2B}$ receptors are present in the human prostrate (see Kursar et al., Mol. Pharmacol. 1994, 46, 227-234) and a receptor with the pharmacological attributes of this receptor subtype mediates contraction of the tissue (see Killam et al., Eur. J. Pharmacol. 1995, 273, 7-14). Some drugs effective in the treatment of benign prostatic hyperplasia block 5-HT mediated contractions of the prostate (see Noble et al., Brit. J. Pharmacol. 1997, 120, 231-238). 5-$HT_{2B}$ receptors mediate smooth muscle and fibrotic hyperplasia (see Launay et al., J. Biol. Chem. 1996, 271, 3141-3147) and serotonin is mitogenic in the prostate (see Cockett et al., Urology 1993, 43, 512-519), therefore a selective 5-$HT_{2B}$ receptor antagonist may have utility not only in mitigating the excessive prostatic constriction, but also in preventing progression of tissue hyperplasia.

Experimental evidence supports a therapeutic role for 5-$HT_{2C}$ receptor antagonists in treating priapism (see Kennett, Curr. Opin. Invest. Drugs 1993, 2, 317-362). MCPP produces penile erections in rats, which effect is blocked by non-selective 5-$HT_{2C/2A}$ receptor antagonists but not by selective 5-$HT_{2A}$ receptor antagonists (see Hoyer, Peripheral actions of 5-HT 1989, Fozard J. (ed.), Oxford University Press, Oxford, 72-99). This therapeutic target for 5-$HT_{2C}$ receptor antagonists is equally a target for 5-$HT_{2B}$ receptor antagonists.

Experimental evidence supports a therapeutic role for histamine $H_1$ receptor antagonists in treating seasonal and perennial allergic rhinitis. The symptomatology of immediate-type allergic diseases, including allergic rhinitis, presumably results from the antigen-induced release of various pharmacologically active substances from mast cells, and from basophilic leukocytes. The substances thus released from these cells, and possibly others as well, are referred to as primary mediators of anaphylaxis and include, among others, histamine. The acute seasonal form of allergic rhinitis, hay fever, and perennial allergic rhinitis are characterized by sneezing, rhinorrhea, nasal congestion, pruritus, conjunctivitis and pharyngitis. In acute seasonal rhinitis, the nose, roof of the mouth, eyes and pharynx often itch, and lacrimation, sneezing and clear, watery nasal discharge follow the pruritus. In perennial rhinitis, chronic nasal obstruction is often prominent and may entend to eustachian tube obstruction. For most patients, topical corticosteroids, some aerosol vasoconstrictor agents, and long acting antihistamine $H_1$ receptor antagonists provide significant relief of symptoms.

Experimental evidence supports a therapeutic role for histamine $H_1$ receptor antagonists in treating allergic pulmonary disease and particular in treating symptoms of allergic bronchial asthma. Patients who suffer from allergic bronchial asthma develop such clinical symptoms as wheezing and dyspnea after exposure to allergens, environmental irritants, viral infections, cold air and exercise. Many of the symptoms result from smooth muscle contraction and vascular dilatation, which, in turn, result from mediator release when the antogen reacts with the IgE antibody on the surface of a mast cell or basophil. This serves as a basis for the use of histamine $H_1$ receptor antagonists.

Experimental evidence supports a therapeutic role for histamine $H_1$ receptor antagonists in treating chronic idiopathic urticaria and some types of physical urticaria. Urticaria is characterized by local wheals and erythema in the dermis; acute urticaria is essentially an anaphylaxis that is limited to the skin and subcutaneous allegy, insect sting, or the like, and is distinct from chronic or idiopathic uricaria which may last several weeks and only rarely be associated with a specific cause. Because these urticarias appear in many cases to be IgE antibody mediated, many of the symptoms may be treated with a $H_1$ receptor antagonist.

In applying the compound of this invention to treatment of the above conditions, administration of the active compound and salts described herein can be via any of the accepted modes of administration, including oral, parenteral

and otherwise systemic route of administration. Any pharmaceutically acceptable mode of administration can be used, including solid, semi-solid or liquid dosage forms, such as, for example, tablets, suppositories, pills, capsules, powders, liquids, suspensions, or the like, preferably in unit dosage forms suitable for single administration of precise dosages, or in sustained or controlled release dosage forms for the prolonged administration of the compound at a predetermined rate. The compositions will typically include a conventional pharmaceutical carrier or excipient and 1-methyl-4-(3-ethoxy-9H-thioxanthene-9-ylidene)-piperidine or the pharmaceutically acceptable salts thereof and, in addition, may include other medicinal agents, pharmaceutical agents, carriers, adjuvants, etc.

The amount of 1-methyl-4-(3-ethoxy-9H-thioxanthene-9-ylidene)-piperidine administered will of course, be dependent on the subject being treated, the severity of the affliction, the manner of administration and the judgment of the prescribing physician. However, an effective dose for oral, parenteral and otherwise systemic routes of administration is in the range of 0.01-20 mg/kg/day, preferably 0.1-10 mg/kg/day. For an average 70 kg human, this would amount to 0.7-1400 mg per day, or preferably 7-700 mg/day.

One of ordinary skill in the art of treating such diseases will be able, without undue experimentation and in reliance upon personal knowledge and the disclosure of this application, to ascertain a therapeutically effective amount of 1-methyl-4-(3-ethoxy-9H-thioxanthene-9-ylidene)-piperidine for a given disease.

For solid compositions, conventional non-toxic solid carriers include, for example, pharmaceutical grades of mannitol, lactose, cellulose, cellulose derivatives, sodium crosscarmellose, starch, magnesium stearate, sodium saccharin, talcum, glucose, sucrose, magnesium carbonate, and the like may be used. The active compound as defined above may be formulated as suppositories using, for example, polyalkylene glycols, acetylated triglycerides and the like, as the carrier. Liquid pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. The composition or formulation to be administered will, in any event, contain a quantity of the active compound(s) in an amount effective to alleviate the symptoms of the subject being treated.

Dosage forms or compositions containing 1-methyl-4-(3-ethoxy-9H-thioxanthene-9-ylidene)-piperidine in the range of 0.25 to 95% by weight with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, cellulose, cellulose derivatives, sodium crosscarmellose, starch, magnesium stearate, sodium saccharin, talcum, glucose, sucrose, magnesium, carbonate, and the like. Such compositions take the form of solutions, suspensions, tablets, pills, capsules, powders, sustained release formulations and the like. Such compositions may contain 1 to 95 % by weight of 1-methyl-4-(3-ethoxy-9H-thioxanthene-9-ylidene)-piperidine, more preferably 2 to 50 % by weight, most preferably 5 to 8 % by weight.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

A more recently devised approach for parenteral administration employs the implantation of a slow-release or sustained-release system, such that a constant level of dosage is maintained (see, e.g., US-A-3,710,795).

The percentage of active compound contained in such parental compositions is highly dependent on the specific nature thereof, as well as the activity of the compound and the needs of the subject. However, percentages of 1-methyl-4-(3-ethoxy-9H-thioxanthene-9-ylidene)-piperidine of 0.1 to 10 % by weight in solution are employable, and will be higher if the composition is a solid which will be subsequently diluted to the above percentages. Preferably the composition will comprise 0.2 to 2 % by weight of 1-methyl-4-(3-ethoxy-9H-thioxanthene-9-ylidene)-piperidine in solution.

In applying the compound of the invention to treatment of diseases or disorders of the eye which are associated with an abnormally high intraocular pressure, administration may be achieved by any pharmaceutically acceptable mode of administration which provides adequate local concentrations to provide the desired response. These include direct administration to the eye via drops and controlled release inserts or implants, as well as systemic administration as previously described.

Drops and solutions applied directly to the eye are typically sterilized aqueous solutions containing 0.1 to 10 % by weight, most preferably 0.5 to 1 % by weight of 1-methyl-4-(3-ethoxy-9H-thioxanthene-9-ylidene)-piperidine,

along with suitable buffer, stabilizer, and preservative. The total concentration of solutes should be such that, if possible, the resulting solution is isotonic with the lacrimal fluid (though this is not absolutely necessary) and has an equivalent pH in the range of pH 6-8. Typical preservatives are phenyl mercuric acetate, thimerosal, chlorobutanol, and benzalkonium chloride. Typical buffer systems and salts are based on, for example, citrate, borate or phosphate; suitable stabilizers include glycerin and polysorbate 80. The aqueous solutions are formulated simply by dissolving the solutes in a suitable quantity of water, adjusting the pH to about 6.8-8.0, making a final volume adjustment with additional water, and sterilizing the preparation using methods known to those in the art.

The dosage level of the resulting composition will, of course, depend on the concentration of the drops, the condition of the subject and the individual magnitude of responses to treatment. However, a typical ocular composition could be administered at the rate of about 2-10 drops per day per eye of a 0.5 % by weight solution of 1-methyl-4-(3-ethoxy-9H-thioxanthene-9-ylidene)-piperidine.

The compositions of the present invention may also be formulated for administration in any convenient way by analogy with other topical compositions adapted for use in mammals. These compositions may be presented for use in any conventional manner with the aid of any of a wide variety of pharmaceutical carriers or vehicles. For such topical administration, a pharmaceutically acceptable non-toxic formulation can take the form of semisolid, liquid, or solid, such as, for example, gels, creams, lotions, solutions, suspensions, ointments, powders, or the like. As an example, the active component may be formulated into a gel using ethanol, propylene glycol, propylene carbonate, polyethylene glycols, diisopropyl adipate, glycerol, water, etc., with appropriate gelling agents, such as Carbomers, Klucels, etc. If desired, the formulation may also contain minor amounts of non-toxic auxiliary substances such as preservatives, antioxidants, pH buffering agents, surface active agents, and the like. Actual methods of preparing such dosage forms are known, or will be apparent, to those skilled in the art; for example, see Remington's Pharmaceutical Sciences, Mack Publishing Company, Easton, Pa., 19th Edition, 1995.

Preferably the pharmaceutical composition is administered in a single unit dosage form for continuous treatment or in a single unit dosage form ad libitum when relief of symptoms is specifically required.

Example

Preparation of 1-Methyl-4-(3-ethoxy-9H-thioxanthene-9-ylidene)-piperidine and corresponding Hydrochloride Salt

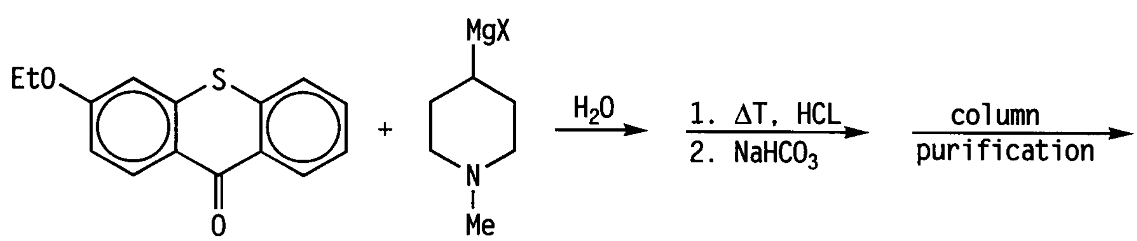


1 2

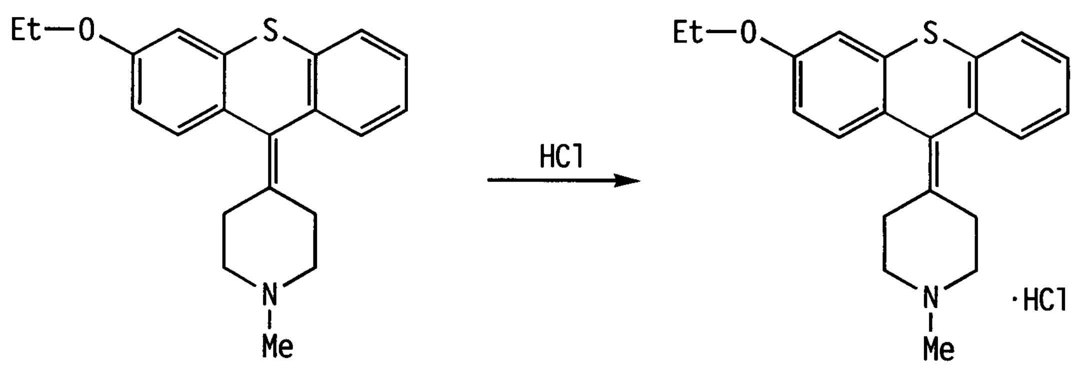


4 10

0.63 g of the 3-ethoxythioxanthene 1 were dissolved in tetrahydrofuran (THF). The Grignard reagent 2 was added to

the solution in one portion. The resulting reaction mixture was stirred under argon at room temperature for 1.5 h until completeness of the reaction. The mixture was quenched with water and extracted three times with ethyl acetate.

The combined organic phase was dried over sodium sulfate, filtered and evaporated to dryness to give about 0.75 g of a brown oil, which was then stirred at reflux for about 50 min in concentrated hydrochloric acid until completeness of the reaction. The mixture was allowed to cool to room temperature and then diluted with water and ethyl acetate. While stirring, small increments of sodium hydrogen carbonate were added until no more gas evolved. The organic layer was collected. The aqueous layer was back extracted five times with ethyl acetate. The combined organic phase was dried over sodium sulfate, filtered and evaporated under vacuum on a rotary evaporator at about 40°C to give about 0.67 g of a brown oil, which was then dissolved in 4 to 6 ml of ethyl acetate and absorbed in 2 to 3 g of silica gel. The silica gel was dried on a rotary evaporator until it was freely flowing as a powder. The dried silica gel was loaded onto a column (about 40 g of silica gel) using 5%-10% methanol/dicloromethane. Desired fractions were combined and evaporated to dryness to give 0.52 g of 1-methyl-4-(3-ethoxy-9H-thioxanthene-9-ylidene)-piperidine 4 as a brown oil (yield = 62%).

In order to prepare the hydrochloride salt 10, the brown oil 4 was dissolved in 3 to 5 ml of ethyl acetate, followed by addition of 4 N hydrochloric acid/dioxane into the stirring solution (3 equivalents of 4 N hydrochloric acid, 0.89 ml). The solution turned brown-red as the hydrochloric acid was added. The mixture was further stirred for 10 min and let settled at room temperature for 20 minutes. 2 to 4 ml of heptane were added to the stirring solution and some red-brown oil separated.

The solvents (heptane und ethyl acetate) were evaporated off. The red-brown oil was chased two times with heptane to get rid off all other solvents. At the second heptane chase some of the oil became solid. It was continued to evaporate the product to dryness to give a red-brown solid (still partially sticky). Heptane was added to the red-brown solid and the product was triturated for 1.5 h at room temperature. Then, the solid was filtered, washed with heptane and dried under vacuum to give 0.51 g of the hydrochloride salt 10 as a red-brown solid (yield = 87%).

Cloned Human 5-$HT_{2B}$ Receptor Binding Assay

The following describes an in vitro binding assay utilizing cloned 5-$HT_{2B}$ receptors radiolabelled with [$^3$H]-5HT.

Receptor Binding Assay

HEK 293 cells transiently transfected with an expression plasmid pXMD1-hu2B encoding the human 5-HT2B receptor (see Schmuck et al., FEBS Lett., 1994, 342, 85-90) were used as described previously (Schmuck et al., Eur. J. Pharmacol., 1996, 8, 959-967). Two days after transfection cells were harvested, pelleted at 500g for 5 min at 4°C, gently resuspended in ice-cold buffer1 (50 mM TRIS pH 7.7, 4 mM $CaCl_2$) and homogenized using a Polytron PT 1200 tissue homogenizer (position 6 for 30 s). Cells were pelleted at 50,000g. 4°C for 10 min, washed with buffer1 and pelleted again. The final pellet was resuspended in incubation buffer (50 mM TRIS pH 7.7, 4 mM $CaCl_2$, 10 μM pargyline and 0.1 % by weight ascorbic acid). The binding assay consisted of 300 μl of membrane suspension (protein concentration = 0.3 to 0.5 mg/ml), 150 μl of competing drug and 50 μl of [$^3$H]5-HT at a final concentration of 4 to 5 nM. The mixture was incubated at 37°C for 30 min and the assay terminated by rapid filtration and two washing steps with 5 ml of cold 20 mM Tris-HCl pH = 7.5, and 154 mM NaCl over Whatman GFB filters. Filters were counted by liquid scintillation. Non-specific binding was determined in the presence of an excess of 5-HT (100 μM). Bound radioligand represented less than 1 % of free radioligand. In competition experiments, specific binding represented about 60 % of total binding. Results are expressed as $pK_i$ values.

The concentration of 1-methyl-4-(3-ethoxy-9H-thioxanthene-9-ylidene)-piperidine producing 50% inhibition of binding ($IC_{50}$) was determined using iterative curve fitting techniques.

Proceeding as in the example above 1-methyl-4-(3-ethoxy-9H-thioxanthene-9-ylidene)-piperidine was found to have affinity for the 5-$HT_{2B}$ receptor.

5-$HT_{2A}$ 5-$HT_{2B}$ 5-$HT_{2C}$ Receptor Binding Methods

The following describes receptor binding methods in which ligands with high affinity for 5-$HT_{2B}$ receptors were counter screened at 5-$HT_{2A}$ and 5-$HT_{2C}$ receptors to demonstrate selectivity.

5-$HT_{2A}$ receptors were labelled with [$^3$H]ketanserin in human cortex, in HEK293 cells expressing a cloned human 5-$HT_{2A}$ receptor and in HEK293 cells expressing the rat 5-$HT_{2A}$ receptor. For competition binding studies the ligand concentration was approximately 0.1 nM. For saturation binding studies concentrations of radioligand ranged from 0.01 nM to 2.0 nM. Assays were conducted in 0.5 ml of assay buffer (50 mM Tris-HCl, 4 mM calcium chloride, 0.1 % by weight ascorbic acid) (pH 7.4 at 4°C). Non-specifc binding was defined with 10 mM unlabelled ketanserin. After a 60 min incubation at 32°C, membranes were harvested onto filters treated with 0.1 % by weight of polyethylenimine and the bound radioactivity was determined.

Human 5-$HT_{2B}$ receptors were labelled in HEK293 cells as described above. except that the radioligand was [$^3$H]-5HT and that the assay buffer contained pargyline in a concentration of 10 mM and 0.1 % by weight of ascorbic acid. For competition binding studies the radioligand concentration was approximately 0.4 nM while for saturation binding studies the concentration of [$^3$H]-5HT ranged from 0.05 to 8 nM. Non-specific binding was defined with 10 mM 5-HT. Incubations were for 120 min at 4°C.

5-$HT_{2C}$ receptors were labelled in choroid plexus, Cos-7 cells expressing the human 5-$HT_{2C}$ receptor and in NIH-3T3 expressing the rat 5-$HT_{2A}$ receptor.

Assays were conducted as described for the 5-$HT_{2A}$ receptor except that the radioligand was [$^3$H]mesulergine. The radioligand concentration for competition studies was approximately 0.2 nM while for saturation binding studies the concentration ranged from 0.1 to 18 nM. Non-specific binding was defined with 10 μM unlabelled mesulergine.

Competition radioligand binding data was analyzed using a four parameter logistic equation and iterative curve-fitting techniques to obtain estimates of the $IC_{50}$ and Hill slope. Kd values, determined from saturation binding studies were then used to calculate inhibition dissociation constants (Ki).

Proceeding as in the example above 1-methyl-4-(3-ethoxy-9H-thioxanthene-9-ylidene)-piperidine was found to have affinity for the 5-$HT_{2B}$ receptor.

5-$HT_{2B}$ Receptor Tissue Based Functional Assay

The following describes an in vitro functional assay characterizing 5-HT receptors (the putative 5-$HT_{2B}$) in rat stomach fundus longitudinal muscle (Baxter et al., Brit. J. Pharmacol. 1994, 112, 323-331).

Strips of longitudinal muscle were obtained from the stomach fundus of male Sprague Dawley rats. The mucosa was removed and the strips were suspended with a resting tension of 1 g in oxygenated (95% $O_2$ /5% $CO_2$) Tyrode solution at 37°C. The composition of the Tyrode solution was as follows (mM): NaCl 136.9; KCl 2.7; $NaH_2PO_4$ 0.4; $MgCl_2$ 1.0; glucose 5.6; $NaHCO_3$ 11.9; $CaCl_2$ 1.8.

Concentation-response curves to 5-HT receptor agonists were constructed under conditions where cyclooxygenase activities were inactivated by 3 μm indomethacin, monoamine oxidase activities inactivated by 0.1 mM pargyline, and uptake mechanisms inactivated by 30 μM cocaine and 30 μM corticosterone.

Effects of drugs were monitored by tension transducers and recorded on polygraph recorders. Tissue response was measured as changes in isometric tension (g). The mean potency ($EC_{50}$) and maximum response were evaluated by standard iterative curve fitting procedures.

Effects of antagonists were determined by measuring dextral shifts to the agonist concentration-response curve after equilibration of the antagonists for at least 1 h. Concentration ratios were measured at half maximal response levels and single concentration antagonist affinities were determined by the equation:

$$KB = \frac{\text{antagonist concentration}}{\text{concentration ratio}}$$

Schild regression analysis was employed with multiple antagonist concentrations when the compound showed competitive behavior.

Proceeding as in the example above, 1-methyl-4-(3-ethoxy-9H-thioxanthene-9-ylidene)-piperidine was found to be an antagonist at the 5-$HT_{2B}$ receptor.

Cloned Human $H_1$, $H_2$, and $H_3$ Receptor Binding Assay

COS-7 cells were transiently transfected with an expression plasmid pCineohH1, pCineohH2, pCineohH3 and pCineohH4 encoding the human Histamine $H_1$, $H_2$ $H_3$ or $H_4$ receptor, respectively. Transfected cells were harvested after 48 h, homogenized in ice-cold 50 mM $Na_2$/potassium phosphate buffer (pH 7.4) and used for radioligand binding studies. Cell homogenates (40 - 50 μg of protein) were incubated for 30 min at 25°C in 50 mM $Na_2$/potassium phosphate buffer (pH 7.4) in 400 μl with the various concentrations of either [$^3$H]-mepyramine, [$^3$H]-thiotidine, [$^3$H]-R-α-Methylhistamine, and [$^3$H]-pyramilamine for cells expressing recombinant human $H_1$, $H_2$, $H_3$ and $H_4$ receptors, respectively. The nonspecific binding was defined in the presence of 1 μM mianserin. In displacement studies, cell homogenated were incubated either with 1 nM [$^3$H]-mepyramine, 15 nM [$^3$H]-thiotidine, 0.5 nM [$^3$H]-R-α-Methylhistamine, or 15 nM [$^3$H]-pyramilamine and increasing concentrations of competing ligands. The incubations were stopped by rapid dilution with 3 ml of ice-cold 50 mM $Na_2$/potassium phosphate buffer (pH 7.4). The bound radioactivity was seperated by filatration through Whatman GF/C filters that had been treated with 0.3 % polyethyleneimine. Filters were washed twice in 3 ml of buffer and radioactivity retained on the filters was measured by liquid scinitllation counting.

The concentration of 1-methyl-4-(3-ethoxy-9H-thioxanthene-9-ylidene)-piperidine producing 50% inhibition of binding

($IC_{50}$) was determined using iterative curve fitting techniques.

Proceeding as in the example above 1-methyl-4-(3-ethoxy-9H-thioxanthene-9-ylidene)-piperidine was found to have affinity for the human $H_1$ receptor.

Human histamine $H_1$ Receptor Functional Assay

For the measurement of the [$^3$H]-inositol phosphate formation transiently transfected HEK-293 cells were seeded in 24 well plates and labeled to equilibrium with *myo*-[2-$^3$H]-inositol (3 ìCi/ml) for an additional 24 hours in growth medium. The medium was aspirated and cells were washed once with 500 µl HBS-buffer (130 mM NaCl, 900 µM $NaH_2PO_4$, 800 µM $MgSO_4$, 5.4 mM KCl, 1.8 mM $CaCl_2$, 25 mM Glucose in 20 mM HEPES pH 7.4). Two min after applying 20 mM $Li^+$ the cells were stimulated by addition of histamine in HBS-buffer. The incubation was stopped by aspiration off the culture medium and the addition of cold 10 mM formic acid. [$^3$H]inositol phosphates were isolated by anion exchange chromatography (see Seuwen et al., 1988, EMBO J., 7, 161-168). The $pK_B$ value was calculated according to the formula: $pK_B$ = log (A'/A - 1) - log [B], where A'/A is the ratio of the agonist concentrations ($EC_{50}$ in the presence / $EC_{50}$ in the absence of antagonist) and [B] the concentration of antagonist.

Proceeding as in the example above 1-methyl-4-(3-ethoxy-9H-thioxanthene-9-ylidene)-piperidine was found to be an antagonist at the human $H_1$ receptor.

## Claims

1. 1-Methyl-4-(3-ethoxy-9H-thioxanthene-9-ylidene)-piperidine according to the Formula

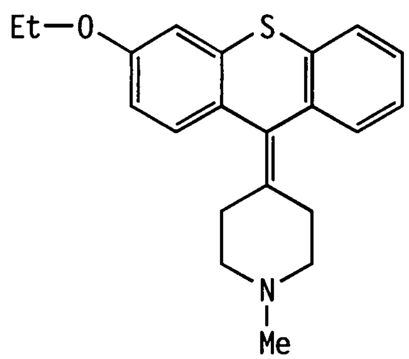

wherein Et represents an ethyl radical and Me represents a methyl radical, or a pharmaceutically acceptable salt thereof.

2. 1-Methyl-4-(3-ethoxy-9H-thioxanthene-9-ylidene)-piperidine for use as a medicament.

3. Use of 1-methyl-4-(3-ethoxy-9H-thioxanthene-9-ylidene)-piperidine for the manufacture of a medicament for treatment of a disease state which is alleviable by treatment with a 5-$HT_{2B}$ antagonist or a H1 antagonist or a mixed 5-$HT_{2b}$/H1 antagonist.

4. The use of claim 3 wherein the disease state is selected from the diseases migraine, asthma, hypertension, restenosis and prostatic hyperplasia or the disease state comprises chronic and / or physical urticaria.

5. The use of claim 3 or 4 wherein the disease state comprises pain.

6. The use of claim 5 wherein the disease state comprises inflammatory pain, neuropathic pain, cancer pain, acute pain or chronic pain.

7. The use of claim 3 wherein the disease state comprises the symptoms of seasonal and perennial allergic rhinitis

8. The use of claim 4 wherein the disease state comprises allergic asthma

**9.** A pharmaceutical composition comprising 1-methyl-4-(3-ethoxy-9H-thioxanthene-9-ylidene)-piperidine or a pharmaceutically acceptable salt thereof, in admixture with one or more pharmaceutically acceptable carriers.

**10.** The pharmaceutical composition of claim 9 additionally comprising at least one further pharmaceutically active compound for pain treatment.

European Patent Office

# EUROPEAN SEARCH REPORT

Application Number
EP 01 11 4643

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | GB 1 000 509 A (SANDOZ AG) 4 August 1965 (1965-08-04) Claims 5 and 7; example 4; page 2, lines 61-64. | 1-10 | C07D409/04<br>A61K31/4436<br>A61P25/06 |
| A,D | US 3 275 640 A (MERCK & CO.) 27 September 1966 (1966-09-27) Claim 2; column 1, lines 34-36. | 1-10 | |
| | | | TECHNICAL FIELDS SEARCHED (Int.Cl.7) |
| | | | C07D<br>A61K<br>A61P |

The present search report has been drawn up for all claims

| Place of search | Date of completion of the search | Examiner |
|---|---|---|
| MUNICH | 9 August 2001 | Weisbrod, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 11 4643

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2001

| Patent document cited in search report | Publication date | Patent family member(s) | Publication date |
|---|---|---|---|
| GB 1000509 A | 04-08-1965 | CH 400152 A | 15-10-1965 |
| | | DE 1470215 A | 08-05-1969 |
| | | FR 2127 M | |
| | | FR 1322527 A | 21-06-1963 |
| | | NL 111765 C | |
| | | SE 313308 B | 11-08-1969 |
| US 3275640 A | 27-09-1966 | CH 429724 A | 15-02-1967 |
| | | DE 1470029 A | 30-04-1969 |
| | | DK 120490 B | 07-06-1971 |
| | | FI 43983 B | 30-04-1971 |
| | | FR 2959 M | |
| | | FR 1501704 A | 31-01-1968 |
| | | GB 1015793 A | |
| | | NL 122808 C | |
| | | NL 283292 A | |
| | | NO 115109 B | 29-07-1968 |
| | | SE 333928 B | 05-04-1971 |

EPO FORM P0459

For more details about this annex : see Official Journal of the European Patent Office, No. 12/82